(19) Europäisches Patentamt
European Patent Office
Office européen des brevets

(11) Publication number: **0 040 916**
**B1**

(12) **EUROPEAN PATENT SPECIFICATION**

(45) Date of publication of patent specification: **12.10.83**

(51) Int. Cl.³: **C 07 C 11/04,**
C 07 C 11/06, C 07 C 7/152

(21) Application number: **81301939.5**

(22) Date of filing: **01.05.81**

(54) **The separation of complexible ligands using alkylation inhibitors.**

(30) Priority: **28.05.80 US 153977**
**23.07.80 US 171631**

(43) Date of publication of application:
**02.12.81 Bulletin 81/48**

(45) Publication of the grant of the patent:
**12.10.83 Bulletin 83/41**

(84) Designated Contracting States:
**BE DE FR GB IT NL**

(56) References cited:
**US - A - 3 758 607**

(73) Proprietor: **Tenneco Resins, Inc.**
**Park 80 Plaza West-1**
**Saddle Brook New Jersey 07662 (US)**

(72) Inventor: **Ostrowski, Paul Charles**
**2838 Pilgrims Point Drive**
**Webster Texas (US)**

(74) Representative: **Oliver, Roy Edward et al,**
**POLLAK,MERCER & TENCH High Holborn House**
**52-54 High Holborn**
**London WC1V 6RY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 040 916 B1

# The separation of complexible ligands using alkylation inhibitors

This invention relates to an improved process for the separation of complexible ligands from gas feedstreams that utilizes complexing of the ligands with liquid sorbents that are solutions of bimetallic salt complexes having the generic formula $M_IM_{II}X_n$·Aromatic, wherein $M_I$ is a Group I—B metal, $M_{II}$ is a Group III—A metal, X is halogen, n is the sum of the valences of $M_I$ and $M_{II}$, and Aromatic is a monocyclic aromatic hydrocarbon or halogenated aromatic hydrocarbon having 6 to 12 carbon atoms. The improvement comprises the inclusion in the liquid sorbents of an amount of an alkylation inhibitor which when dissolved in the sorbents inhibits the alkylation of their aromatic component by the lower olefins that are present in the gas feedstream.

Bimetallic salt complexes that have the generic formula $M_IM_{II}X_n$·Aromatic are known to be useful in the separation from gas mixtures of such complexible ligands as olefins, acetylenes, aromatics, and carbon monoxide. For example, in U.S. patent No. 3,651,159, Long et al. disclosed a process in which a sorbent solution of cuprous aluminum tetrahalide in toluene was used to separate ethylene, propylene, and other complexible ligands from a gas feedstream. The complexed ligands were recovered by ligands exchange with toluene. The resulting solution of cuprous aluminium tetrahalide·toluene in toluene was recycled and used to separate additional quantities of the complexible ligand from the gas feedstream. Walker et al in U.S. patent No. 3,647,843 disclosed a process in which a hydrocarbon pyrolysis gas feedstream was contacted with a cuprous aluminium tetrachloride solution in toluene to separate acetylene from the gas feedstream as a solution of the complex $HC\equiv CH\cdot CuAlCl_4$ in toluene. Aceytlene was stripped from this complex, and the cuprous aluminum tetrachloride·toluene solution was recycled.

In processes such as those disclosed by Long et al. and by Walker et al. in which a liquid sorbent containing a bimetallic salt complex is recycled without purification and is used for long periods of time, there is a gradual increase in the amounts of reaction by-products and other impurities in it until sufficient impurities are present to interfere with the efficient operation of the process. For example, when the liquid sorbent is contacted with a gas stream that contains ethylene and/or propylene, some of the olefin reacts with the aromatic hydrocarbon or halogenated aromatic hydrocarbon in the sorbent to form alkylated aromatic compounds and some undergoes polymerization to form olefin oligomers. These reactions are catalyzed by hydrogen chloride or other acidic compounds that are in the gas feedstream or are formed as by-products of the reaction between the liquid sorbent and trace amounts of water or certain other impurities in the gas feedstream.

In ligand separation processes that involve the complexing of ligands with a liquid sorbent that is a solution of a bimetallic salt complex in an aromatic hydrocarbon, it is necessary to minimize the formation of alkylated aromatic compounds because the presence of these compounds not only adversely affects the complexing ability of the liquid sorbent, but it also leads to corrosion of the processing equipment and copper metal deposition.

A number of procedures have been proposed in the prior art for inhibiting the reactions between the liquid sorbent and lower olefins to form alkylated aromatic compounds and olefin oligomers by removing or neutralizing the acidic materials that catalyze these reactions, but none has proven to be entirely satisfactory. Some of these procedures fail to reduce the amounts of reaction by-products to the desired very low levels, while others interfere with the efficient operation of the ligand separation process. For example, Long et al. in U.S. patents No. 3,651,195, No. 3,887,600, No. 4,066,679, and No. 4,141,960 disclosed the use of a small amount of a neutralizing agent, such as ammonia or an organic nitrogen compound, to reduce the residual catalytic activity or acidity of the system. They disclosed that the amount of neutralizing agent should be merely enough to react with the free acidity of the system, because larger amounts of the neutralizing agent will cause precipitation of the copper salt from the solution and lead to the formation of different catalytic species. They preferred to use from 0.01 to 1 wt. percent, based on the liquid sorbent, of the neutralizing agent. Combinations of organic phosphines and organic nitrogen bases are described in U.S. Patent No. 3,785,609 as serving to inhibit side reactions during olefin complexing processes in which liquid sorbents containing cuprous aluminium tetrachloride are used as the complexing agent. The useful organic nitrogen bases include substituted pyridines, tertiary alkyl amines and tertiary alkyl aryl amines. Pyridine is said to be ineffective as an inhibitor because it reacts with the liquid sorbent to form precipitates which contain sizeable amounts of the organic base. U.S. Patents No. 3,755,487 and 3,758,608 describe the addition of soluble compounds of antimony, arsenic and bismuth, phosphines, amines and other additives to liquid sorbents which comprise cuprous aluminium tetrachloride, in order to minimise side reactions, to reduce the corrosion effect of the cuprous salt solution and to prevent the deposition of copper from the solution. U.S. Patents Nos. 3,776,972 and 3,933,878 disclose that trialkyl phosphines and other complexible ligands can be used to inhibit alkylation and polymerization side reactions in olefin-complexing processes employing liquid sorbents which comprise cuprous aluminium tetrachloride and an aromatic hydrocarbon.

In accordance with this invention, a process is provided for the separation of complexible ligands from a gas feedstream which comprises ethylene, propylene or mixtures thereof, wherein (a) the gas feedstream is contacted with a liquid sorbent comprising a solution in an aromatic hydrocarbon or

halogenated aromatic hydrocarbon of a bimetallic salt complex having the formula $M_IM_{II}X_n$. Aromatic, wherein $M_I$ is a Group I—B metal, $M_{II}$ is a Group III—A metal, X is halogen, n is the sum of the valences of $M_I$ and $M_{II}$ and Aromatic is an aromatic hydrocarbon or halogenated aromatic hydrocarbon having 6 to 12 carbon atoms, thereby forming a reaction mixture which comprises a solution of a complex of the complexible ligand and the bimetallic salt complex in the liquid sorbent, (b) the reaction mixture is separated from the gas feedstream, (c) the ligand is separated from the liquid sorbent in the reaction mixture and (d) the liquid sorbent is recycled to Step (a), characterized in that the liquid sorbent contains from 0.1 to 20 mole percent, based on the Group I—B metal in the bimetallic salt complex component of the liquid sorbent, of an alkylation inhibitor comprising zinc diphenyl or triphenylboron.

The presence of the alkylation inhibitor in the liquid sorbent makes it possible to absorb ethylene and/or propylene reversibly without encountering appreciable deterioration of the liquid sorbent resulting from reaction between the liquid sorbent and the olefins, thereby lengthening the time that the liquid sorbent can be used without purification in the ligand separation process.

The liquid sorbents stabilized by the process of this invention are solutions of a bimetallic salt complex in an aromatic hydrocarbon or a halogenated aromatic hydrocarbon. The bimetallic salt complexes have the generic formula $M_IM_{II}X_n$·Aromatic. $M_I$ is a Group I—B metal; that is, copper, silver, or gold. Copper (I) is the preferred metal. $M_{II}$ is a Group III—A metal; that is boron, aluminium, gallium, indium or thallium. Boron and aluminium are the preferred metals, aluminium being particularly preferred. X is halogen, i.e., fluorine, chlorine, bromine or iodine; it is preferably chlorine or bromine. The sum of the valences of $M_I$ and $M_{II}$ is represented by n. Aromatic is a monocyclic aromatic hydrocarbon or halogenated aromatic hydrocarbon having 6 to 12 carbon atoms, preferably 6 to 9 carbon atoms, such as benzene, toluene, ethylbenzene, xylene, mesitylene, chlorobenzene, bromobenzene, iodobenzene, dichlorobenzene, dibromobenzene, chlorotoluene, bromotoluene, iodotoluene or chloroxylene. It is preferably benzene or toluene. Illustrative of these bimetallic salt complexes are the following: $CuBF_4$·benzene, $CuBCl_4$·benzene, $AgBF_4$·mesitylene, $AgBCl_4$·xylene, $AgAlCl_4$·xylene, $AgAlBr_4$·bromobenzene, $CuGaCl_4$·toluene, $CuInI_4$·1,2-dichlorobenzene, $CuTlI_4$·p-chlorotoluene, and the like. In accordance with a preferred feature of the process of the invention, the liquid sorbent is a solution in an aromatic hydrocarbon or halogenated aromatic hydrocarbon of the bimetallic salt complex having the formula $CuAlCl_4$·Aromatic. The preferred bimetallic salt complexes are $CuAlCl_4$·benzene, $CuAlCl_4$·toluene, and $CuAlBr_4$·benzene. The aromatic hydrocarbon or halogenated aromatic hydrocarbon in which the bimetallic salt complex is dissolved is usually and preferably the same as that used in the preparation of the bimetallic salt complex, but if desired it may be a different one. The total amount of aromatic hydrocarbon or halogenated aromatic hydrocarbon in the liquid sorbent, that is, the amount in the bimetallic salt complex plus the amount used as solvent, is at least 10 mole percent of the amount of the bimetallic salt $M_IM_{II}X_n$ that is present. It is preferred that the amount of aromatic hydrocarbon or halogenated aromatic hydrocarbon be 100 to 450 mole percent of the amount of the bimetallic salt. The particularly preferred liquid sorbents contain 25 to 75 percent by weight of $CuAlCl_4$·benzene in benzene or $CuAlCl_4$·toluene in toluene.

In the practice of this invention, a gas feedstream that contains ethylene, propylene, or a mixture thereof is contacted with a liquid sorbent that contains an alkylation-inhibiting amount of either zinc diphenyl or triphenylboron. When the gas feedstream is contacted with a sorbent that contains zinc diphenyl as the alkylation inhibitor, any water that is in the gas feedstream reacts with the zinc diphenyl to form zinc oxide and benzene, as is shown in the following equation:

1) $Zn(C_6H_5)_2 + H_2O \rightarrow ZnO + 2C_6H_6$

rather than with the cuprous aluminum tetrachloride in the liquid sorbent by the reactions shown in the following equations:

2) $2Cu\ AlCl_4 \cdot toluene + H_2O \rightarrow HCl + CuCl + CuAlCl_4 \cdot Al(OH)Cl_2 \cdot toluene$

3) $CuAlCl_4 \cdot Al(OH)Cl_2 \cdot toluene \rightarrow HCl + CuAlCl_4 \cdot Al\ O\ Cl \cdot toluene$

4) $2\ CuAlCl_4 \cdot Al\ O\ Cl \cdot toluene\ (solid) + toluene \xrightarrow{\Delta} CuAlCl_4 \cdot toluene + Al\ O\ Cl + CuAlCl_4 \cdot Al\ O\ Cl \cdot toluene\ (liquid)$

which yield as a reaction by-product hydrogen chloride, which catalyzes alkylation and other side reactions. When the gas feedstream is contacted with a liquid sorbent that contains triphenylboron as the alkylation inhibitor, any water that is in the gas feedstream reacts with the cuprous aluminium tetra-chloride in the liquid sorbent to form hydrogen chloride as a reaction by-product, as is shown in equations 2) and 3). The hydrogen chloride that is formed then reacts with the triphenylboron to form benzene and boron trichloride which can readily be removed from the liquid sorbent. The reaction between triphenylboron and hydrogen chloride is shown in the following equation:

5) $(C_6H_5)_3B + 3\ HCl \rightarrow B\ Cl_3 + 3\ C_6H_6$

The amount of the alkylation inhibitor incorporated in the liquid sorbent is at least the amount required to remove from the gas feedstream the traces of water and other impurities which yield hydrogen chloride and other acidic compounds when they react with the bimetallic salt complex in the sorbent or to react with the hydrogen chloride and other acidic compounds which are formed when the traces of water and certain other impurities in the gas feedstream react with the bimetallic salt complex in the liquid sorbent. The alkylation inhibitor is used in an amount from 0.1 mole percent to 20 mole percent, based on the copper or other Group I—B metal in the bimetallic salt complex. Preferably from 1 mole percent to 10 mole percent of the inhibitor is used to inhibit the side reactions. When the alkylation inhibitor is triphenyl boron, best results are obtained when from 2 mole percent to 5 mole percent of the inhibitor is used.

In the practice of this invention, all of the alkylation inhibitor may be added to the liquid sorbent before the sorbent is contacted with the gas feedstream, or a minor portion (less than 50%) of the inhibitor may be present at the start of the ligand-separation process and the remainder added continuously or intermittently during the ligand-separation process at approximately the rate at which it is being removed from the liquid sorbent by the reaction with water, hydrogen chloride, or other impurities in the gas feedstream.

Either zinc diphenyl, triphenylboron, or a solution of either of these inhibitors in a liquid aromatic hydrocarbon or halogenated aromatic hydrocarbon may be added to the liquid sorbent. The inhibitor is preferably added as a saturated solution in benzene or toluene.

The zinc diphenyl and the triphenylboron that are used to stabilize the liquid sorbent by inhibiting alkylation of the aromatic compounds in it may be prepared by any suitable and convenient procedure. For example, zinc diphenyl can be prepared by the Grignard reaction from zinc chloride and phenyl magnesium bromide. After separation from the reaction mixture and vacuum distillation to remove etherates of zinc diphenyl and other volatile reaction by-products, the product is preferably dissolved in toluene to form a saturated 0.46 M solution of zinc diphenyl in toluene. Triphenylboron can be prepared by the Grignard reaction from phenyl magnesium bromide and boron trifluoride etherate. This procedure was described in detail by Krause and Nitsche in Ber. *55,* 1261 (1922) and by Krause and Polak in Ber. *59,* 777 (1926). Alternatively, it can be prepared by the procedure described by Wittig and Raff in Ann. *573,* 195 (1951), which is shown in equation 6):

$$6)\ NaB\Phi_4 \xrightarrow[H_2O]{(CH_3)_3N\cdot HCl} [(CH_3)_3NH]^+[B\Phi_4]^- \xrightarrow[N_2]{200^\circ C.} \Phi H + (CH_3)_3N + \Phi_3B$$

Because both zinc diphenyl and triphenylboron reaction readily with oxygen, air must be excluded from the compounds, from their solutions, and from liquid sorbents that contain them.

This procedure for the stabilization of liquid sorbents by inhibiting alkylation of the aromatic compounds in the sorbent is useful not only in processes in which ethylene and/or propylene is being separated from gas feedstreams but also in those in which carbon monoxide or another complexible ligand is being separated from a gas feedstream that contains trace amounts of the lower olefins as impurities.

The invention is further illustrated by the following examples.

## Example 1

A. A liquid sorbent that contained 28.6 mole percent of cuprous aluminum tetrachloride and 71.4 mole percent of toluene was prepared by adding 1.1 moles of cuprous chloride to 1 mole of anhydrous aluminum chloride in toluene. The resulting solution was filtered to remove unreacted cuprous chloride and insoluble impurities from it.

B. To the liquid sorbent was added a sufficient amount of a saturated solution of zinc diphenyl in toluene to form a solution that contained 7.36 mole percent of zinc based on copper in the liquid sorbent.

C. The zinc diphenyl-containing liquid sorbent was contacted with propylene at 65°C. at a pressure of 450 torr for 67 hours. The resulting liquid sorbent that contained the propylene-cuprous aluminum tetrachloride complex was fed to a stripping column in which it was brought into contact with benzene vapor at 95°C. The mixture of benzene vapor and olefins that left the column was cooled to separate the benzene from the olefins. The stripped liquid sorbent was analyzed to determine the amount of alkylation that had taken place. The results obtained are summarized in Table I.

From the data in Table I, it will be seen that during the process in which propylene was contacted with a liquid sorbent that comprised cuprous aluminum tetrachloride and toluene for 67 hours, the alkylation of toluene was substantially prevented by the addition of a small amount of zinc diphenyl to the liquid sorbent.

## Comparative Example A

The procedure described in Example 1 was repeated except that zinc diphenyl was not added to the liquid sorbent before the sorbent was contacted with propylene and that the sorbent was contacted

with propylene for only 0.5 hour. After the complexed propylene had been removed from it, the stripped liquid was analyzed to determine the amount of alkylation that had taken place. The results obtained are summarized in Table I.

TABLE I

Analysis of Inhibited and Uninhibited Liquid Sorbent After Contact With Propylene at 65°C. at 450 Torr

| | Liquid Sorbent containing 7.36 mole pecent of Zinc Diphenyl | Uninhibited Liquid Sorbent |
|---|---|---|
| Time of Contact with Propylene (Hours) | 67 | 0.5 |
| *Analysis* (mmol) | | |
| Monoisopropyltoluene | 0.095 | 23.40 |
| Diisopropyltoluene | < 0.001 | 7.15 |
| Triisopropyltoluene | < 0.001 | 0.038 |
| Total isopropyl groups | 0.095 | 37.81 |
| Copper (total) | 20.3 | 31.0 |
| Copper metal | 0.301 | —— |
| Ratio — isopropyl/copper | 0.0047 | 1.220 |

From the data in this table, it will be seen that the toluene in the uninhibited liquid sorbent underwent considerable alkylation when the liquid sorbent was contactd with propylene.

Example 2

To a liquid sorbent prepared by the procedure of Example 1A that contained 6.760 parts by weight (16.3 mmol) of cuprous aluminum tetrachloride and 32.2 mmol of toluene was added 0.469 part by weight (2.14 mmol) of zinc diphenyl under nitrogen. After the liquid sorbent had been heated to 55°C., 8.64 mmol of propylene was added to it. After 19.1 hours at 55°C., the reaction mixture was vacuum stripped twice to yield 7.68 mmol of gas that contained 7.43 mmol of propylene and 0.069 mmol of propane.

Example 3

To a liquid sorbent prepared by the procedure of Example 1A that contained 6.760 parts by weight (16.3 mmol) of cuprous aluminum tetrachloride and 32.2 mmol of toluene was added 0.469 part by weight (2.14 mmol) of zinc diphenyl under nitrogen. After the sorbent had been heated to 84°C., 7.70 mmol of propylene was added to it. The reaction mixture was heated to 84°C. for 23 hours and then vacuum stripped twice to yield a gas that contained 6.83 mmol of propylene, 0.002 mmol of hexenes, 0.022 mmol of benzene, and 0.547 mmol of toluene.

Example 4

A. To a 5 ml. portion of a liquid sorbent prepared by the procedure of Example 1A that contained 14.4 mmol of cuprous aluminum tetrachloride and 34 mmol of toluene was added 1 ml. of a saturated zinc diphenyl solution in toluene (0.46 mmol zinc diphenyl and 9.2 mmol toluene). The liquid sorbent was heated to 80°C. and 4.41 mmol of ethylene was added to it. After 17 hours at 80°C., the reaction

mixture was stripped under vacuum. The recovered gas contained 99.9% of the ethylene that had been added to the liquid sorbent.

B. To the liquid sorbent from which ethylene had been recovered in Example 4A was added 28 mmol of toluene. The resulting liquid sorbent was heated to 84°C. and 3.07 mmol of propylene was added to it. After 17 hours at 84°C., the reaction mixture was stripped under vacuum and then cooled to 25°C. An additional 6.56 mmol of propylene was added to the liquid sorbent. After 2.5 hours at 25°C., the reaction mixture was stripped under vacuum. The gas that was recovered contained 94.8% of the propylene that had been added to the liquid sorbent.

Example 5

When the procedure described in Example 4 was repeated except that a saturated solution of zinc diphenyl in benzene was added to a liquid sorbent that was a solution of cuprous aluminum tetrachloride in benzene, similar results were obtained.

Example 6

A. To a 5 ml. portion of a liquid sorbent prepared by the procedure of Example 1A that contained 13.8 mmol of cuprous aluminum tetrachloride was added 3 ml. of a saturated zinc diphenyl solution in toluene to form a liquid sorbent that contained 1.26 mmol of zinc diphenyl (9.1 mole % of zinc diphenyl based on the copper in the liquid sorbent). The liquid sorbent was heated to 80°C., and 2.66 mmol of propylene was added to it. After 17 hours at 80°C., the reaction mixture was stripped under vacuum. The gas that was recovered contained 95.8% propylene, 3.6% propane and 0.6% ethane. The residual liquid sorbent was found to contain 0.326 mmol of benzene, 25.1 mmol of toluene, and 0.015 mmol of isopropyl-toluenes.

B. The residual liquid sorbent was mixed with 3 ml. of fresh toluene, complexed with 2.85 mmol of propylene at 80°C. for 19 hours, and then vacuum stripped. The gas that was recovered contained 96.2% of propylene, 3.3% of propane, and 0.3% of ethane. The second residual liquid sorbent contained 22.7 mmol of toluene, 0.065 mmol of benzene, and 0.013 mmol of isopropyltoluenes.

C. The second residual liquid sorbent was mixed with 3 ml. of fresh toluene, complexed with 3.31 mmol of propylene at 80°C. for 17 hours, and then vacuum stripped. The gas that was recovered contained 96.7% of propylene, 3.1% of propane, and 0.2% of ethane. The third residual liquid sorbent contained 22.5 mmol of toluene, 0.189 mmol of benzene, and 0.013 mmol of isopropyltoluenes.

D. The third residual liquid sorbent was mixed with 3 ml. of fresh toluene, complexed with 3.43 mmol of propylene at 80°C. for 16.7 hours, and then vacuum stripped. The gas that was recovered contained 96.4% of propylene, 3.3% of propane and 0.2% of ethane. The fourth residual liquid sorbent contained 20.2 mmol of toluene, 0.16 mmol of benzene, and 0.038 mmol of isopropyltoluenes.

During this 4-cycle process, 85% of all of the propylene added to the zinc diphenyl-containing liquid sorbent was recovered in the gas that was stripped from the liquid sorbent containing the propylene-cuprous aluminum tetrachloride complex. At the same time, about 30% of the sinc diphenyl reacted with water that was in the propylene added to the liquid sorbent to form zinc oxide and benzene.

Example 7

A. A solution of cuprous aluminum tetrachloride in toluene was prepared by adding 1.1 moles of cuprous chloride to 1 mole of anhydrous aluminum chloride in toluene. The solution was filtered to remove unreacted cuprous chloride and insoluble impurities from it and then heated under vacuum to separate toluene and other volatile materials from the cuprous aluminum tetrachloride. The cuprous aluminum tetrachloride was dissolved in fresh toluene to form a liquid sorbent that had a density of 1.393 g./ml. and that contained 36.5 mmol of copper per 10 ml. of sorbent.

B. To a 10 ml. portion of the liquid sorbent was added a solution of 226 mg. of triphenylboron in 2 ml. of toluene to form a solution that contained 2.56 mole percent of triphenylboron, based on copper in the liquid sorbent.

C. The triphenylboron-containing liquid sorbent was contacted with propylene at 65°C. at an initial pressure of 720 torr for 3 hours. The final pressure was 370 torr, which was reached after about 15 minutes. The resulting liquid sorbent that contained the propylene-cuprous aluminum tetrachloride complex was stripped under vacuum to remove the propylene from it. The stripped liquid sorbent was then analyzed to determine the amount of alkylation that had taken place. The results obtained are summarized in Table II.

D. For comparative purposes, 10 ml. of the liquid sorbent that did not contain triphenylboron was contacted with propylene at 65°C. at an initial pressure of 720 torr for 0.5 hour. The resulting liquid sorbent that contained the propylene-cuprous aluminum tetrachloride complex was stripped under vacuum to remove the propylene from it, and the stripped liquid sorbent was analyzed to determine the amount of alkylation that had taken place. The results obtained are summarized in Table II.

TABLE II

Analysis of Inhibited and Uninhibited Liquid Sorbent After Contact With Propylene at 65°C.

| | Liquid Sorbent Containing 2.56 mole percent of Triphenylboron | Uninhibited Liquid Sorbent |
|---|---|---|
| Time of Contact with Propylene (Hours) | 3 | 0.5 |
| *Analysis* (mmol) | | |
| Monoisopropyltoluene | 0.00125 | 0.755 |
| Diisopropyltoluene | < 0.00001 | 0.231 |
| Triisopropyltoluene | < 0.00001 | 0.001 |
| Ratio — isopropyl/copper | 0.00125 | 1.220 |

From the data in Table II, it will be seen that during the process in which propylene was contacted with a liquid sorbent that comprised cuprous aluminum tetrachloride and toluene, the alkylation of toluene was substantially inhibited by the addition of a small amount of triphenylboron to the liquid sorbent. The calculated inhibition factor is 5830.

Example 8

A. A solution of cuprous aluminum tetrachloride in benzene was prepared by adding 1.1 moles of cuprous chloride to 1 mole of anhydrous aluminum chloride in benzene. The solution was filtered to remove unreacted cuprous chloride and insoluble impurities from it. The resulting liquid sorbent had a density of 1.204 g./ml., and it contained 20.0 mmol of copper per 10 ml. of sorbent.

B. To a 10 ml. portion of the liquid sorbent was added a solution of 143.5 mg. of triphenylboron in 1 ml. of benzene to form a solution that contained 2.96 mole percent of triphenylboron, based on copper in the liquid sorbent.

C. The triphenylboron-containing liquid sorbent was contacted with ethylene at 65°C. for 3 hours at an initial pressure of 720 torr. The resulting liquid sorbent that contained the ethylene-cuprous aluminum tetrachloride complex was stripped under vacuum to remove the ethylene from it. The stripped liquid sorbent was then analyzed to determine the amount of alkylation that had taken place. The results obtained are summarized in Table III.

D. For comparative purposes, 10 ml. of the liquid sorbent that did not contain triphenylboron was contacted with ethylene at 65°C. for 3 hours at an initial pressure of 720 torr. The resulting liquid sorbent was then stripped under vacuum to remove the ethylene from it and the stripped sorbent was analyzed to determine the amount of alkylation that had taken place. The results obtained are summarized in Table III.

TABLE III

Analysis of Inhibited and Uninhibited Liquid Sorbent After Contact With Ethylene at 65°C.

| | Liquid Sorbent Containing 2.96 mole percent of Triphenylboron | Uninhibited Liquid Sorbent |
|---|---|---|
| *Analysis* (mmol) | | |
| Monoethylbenzene | 0.00796 | 0.0642 |
| Diethylbenzene | 0.00076 | 0.00191 |
| Triethylbenzene | 0.00067 | 0.00045 |
| Pentaethylbenzene | 0.00014 | 0.00013 |
| Hexaethylbenzene | 0.00039 | 0.00097 |
| 1,1-Diphenylethane | 0.00131 | 0.00076 |
| Ratio — Ethyl/copper | 0.00129 | 0.06169 |

The data in Table III demonstrate that during the process in which ethylene was contacted with a liquid sorbent that comprised cuprous aluminum tetrachloride and benzene, the alkylation of benzene was substantially inhibited by the presence of a small amount of triphenylboron in the liquid sorbent.

Example 9

To 5 ml. of a liquid sorbent prepared by the procedure of Example 7A that had a density of 1.31 g./ml. and contained 14.3 mmol of cuprous aluminum tetrachloride was added 3 ml. of an 0.155 M triphenylboron solution in toluene. The resulting triphenylboron-containing sorbent was contacted with 3.6 mmol of ethylene at 80°C. for 3 hours. When the resulting reaction mixture was stripped twice under vacuum, 3.5 mmol of ethylene was recovered.

**Claims**

1. A process for the separation of complexible ligands from a gas feedstream which comprises ethylene, propylene or mixtures thereof, wherein (a) the gas feedstream is contacted with a liquid sorbent comprising a solution in an aromatic hydrocarbon or halogenated aromatic hydrocarbon of a bimetallic salt complex having the formula $M_IM_{II}X_n\cdot$Aromatic, wherein $M_I$ is a Group I—B metal, $M_{II}$ is a Group III—A metal, X is halogen, n is the sum of the valences of $M_I$ and $M_{II}$ and Aromatic is an aromatic hydrocarbon or halogenated aromatic hydrocarbon having 6 to 12 carbon atoms, thereby forming a reaction mixture which comprises a solution of a complex of the complexible ligand and the bimetallic salt complex in the liquid sorbent, (b) the reaction mixture is separated from the gas feedstream, (c) the ligand is separated from the liquid sorbent in the reaction mixture and (d) the liquid sorbent is recycled to Step (a), characterized in that the liquid sorbent contains from 0.1 to 20 mole percent, based on the Group I—B metal in the bimetallic salt complex component of the liquid sorbent, of an alkylation inhibitor comprising zinc diphenyl or triphenylboron.

2. A process according to claim 1, wherein the liquid sorbent is a solution in an aromatic hydrocarbon or halogenated aromatic hydrocarbon of the bimetallic salt complex having the formula $CuAlCl_4\cdot$Aromatic.

3. A process according to claim 2, wherein the liquid sorbent is a solution of $CuAlCl_4$-toluene in toluene.

4. A process according to claim 2, wherein the liquid sorbent is a solution of $CuAlCl_4\cdot$benzene in benzene.

5, A process according to any of claims 1 to 4, wherein the liquid sorbent contains from 1 to 10 mole precent, based on the Group I—B metal in the bimetallic salt complex, of zinc diphenyl.

6. A process according to any of claims 1 to 4, wherein the liquid sorbent contains from 1 to 10 mole percent, based on the Group I—B metal in the bimetallic salt complex, of triphenylboron.

7. A process according to claim 6, wherein the liquid sorbent contains from 2 to 5 mole percent, based on the copper in the bimetallic salt complex, of triphenylboron.

8. A process according to any of claims 1 to 7, wherein less than 50% of the zinc diphenyl or triphenylboron is present at the start of the ligand separation process and the remainder is added intermittently or continuously during the ligand separation process.

## Revendications

1. Procédé pour séparer des ligands aptes à la complexation d'une charge de gaz qui comprend de l'éthylène, du propylène ou leurs mélanges, dans lequel (a) la charge de gaz est mise en contact avec un produit sorbant liquide comprenant une solution dans un hydrocarbure aromatique ou dans un hydrocarbure aromatique halogéné, d'un sel complexe bimétallique de formule $M_IM_{II}X_n$·Aromatique dans lequel $M_I$ est un métal du Groupe I—B, $M_{II}$ est un métal du Groupe III—A, X est un halogène, *n* est la somme des valences de $M_I$ et $M_{II}$ et Aromatique désigne un hydrocarbure aromatique ou un hydrocarbure aromatique halogéné ayant 6 à 12 atomes de carbone, de manière à former un mélange réactionnel qui comprend une solution d'un complexe du ligand apte à la complexation et du sel complexe bimétallique dans le produit sorbant liquide, (b) le mélange réactionnel est séparé de la charge gazeuse, (c) le ligand est séparé du produit sorbant liquide dans le mélange réactionnel et (d) le produit sorbant liquide est recyclé dans l'étape (a), caractérisé en ce que le produit sorbant liquide contient 0,1 à 20 moles %, sur la base du métal du Groupe I—B du sel complexe bimétallique du produit sorbant liquide, d'un inhibiteur d'alkylation comprenant du zinc-diphényle ou du triphénylbore.

2. Procédé suivant la revendication 1, dans lequel le produit sorbant liquide est une solution d'un hydrocarbure aromatique ou d'un hydrocarbure aromatique halogéné du sel complexe bimétallique de formule $CuAlCl_4$·Aromatique.

3. Procédé suivant la revendication 2, dans lequel le produit sorbant liquide est une solution de $CuAlCl_4$-toluène dans le toluène.

4. Procédé suivant la revendication 2, dans lequel le produit sorbant liquide est une solution de $CuAlCl_4$·benzène dans le benzène.

5. Procédé suivant l'une quelconque des revendications 1 à 4, dans lequel le produit sorbant liquide contient 1 à 10 moles %, sur la base du métal du Groupe I—B du sel complexe bimétallique, de zinc-diphényle.

6. Procédé suivant l'une quelconque des revendications 1 à 4, dans lequel le produit sorbant liquide contient 1 à 10 moles %, sur la base du métal du Groupe I—B du sel complexe bimétallique, de triphénylbore.

7. Procédé suivant la revendication 6, dans lequel le produit sorbant liquide contient 2 à 5 moles %, sur la base du cuivre contenu dans le sel complexe bimétallique, de triphénylbore.

8. Procédé suivant l'une quelconque des revendications 1 à 7, dans lequel moins de 50% du zinc-diphényle ou du triphénylbore sont présents au début de l'opération de séparation des ligands et le reste est ajouté par intermittence ou continuellement pendant l'opération de séparation des ligands.

## Patentansprüche

1. Ein Verfahren zur Abtrennung von komplexierbaren Liganden aus einem gasförmigen Beschickungsstrom, der Äthylen, Propylen oder Gemische davon umfaßt, wobei

(a) der gasförmige Beschickungsstrom mit einem flüssigen Sorptionsmittel in Berührung gebracht wird, das eine Lösung eines Bimetall-Salzkomplexes der Formel $M_IM_{II}X_n$·Aromat, wobei $M_I$ ein Metall der Gruppe I—B, $M_{II}$ ein Metall der Gruppe III—A und X ein Halogenatom bedeuten, n die Summe der Valenzen von $M_I$ und $M_{II}$ ist und Aromat einen aromatischen Kohlenwasserstoff oder einen halogenierten aromatischen Kohlenwasserstroff mit 6 bis 12 Kohlenstoffatomen darstellt, in einem aromatischen Kohlenwasserstoff oder halogenierten aromatischen Kohlenwasserstoff umfaßt, wobei ein Reaktionsgemisch entsteht, das eine Lösung eines Komplexes des komplexierbaren Liganden und des Bimetall-Salzkomplexes in dem flüssigen Sorptionsmittel enthält,

(b) das Reaktionsgemisch von dem gasförmigen Beschickungsstrom abgetrennt,

(c) der Ligand von dem flüssigen Sorptionsmittel in dem Reaktionsgemisch abgetrennt und

(d) das flüssige Sorptionsmittel in die Stufe (a) zurückgeführt wird,

dadurch gekennzeichnet, daß das flüssige Sorptionsmittel 0,1 bis 20 Molprozent, bezogen auf das Metall der Gruppe I—B in der Bimetall-Salzkomplex-Komponente des flüssigen Sorptionsmittels, eines Zinkdiphenyl oder Triphenylbor umfassenden Alkylierungsinhibitors enthält.

2. Ein Verfahren nach Anspruch 1, wobei das flüssige Sorptionsmittel eine Lösung des Bimetall-Salzkomplexes der Formel $CuAlCl_4$·Aromat in einem aromatischen Kohlenwasserstoff oder einem halogenierten aromatischen Kohlenwasserstoff ist.

3. Ein Verfahren nach Anspruch 2, wobei das flüssige Sorptions mittel eine Lösung von $CuAlCl_4$·Toluol in Toluol ist.

4. Ein Verfahren nach Anspruch 2, wobei das flüssige Sorptionsmittel eine Lösung von $CuAlCl_4 \cdot$Benzol in Benzol ist.

5. Ein Verfahren nach einem der Ansprüche 1 bis 4, wobei das flüssige Sorptionsmittel 1 bis 10 Molprozent, bezogen auf das Metall der Gruppe I—B in dem Bimetall-Salzkomplex, Zinkdiphenyl enthält.

6. Ein Verfahren nach einem der Ansprüche 1 bis 4, wobei das flüssige Sorptionsmittel 1 bis 10 Molprozent, bezogen auf das Metall der Gruppe I—B in dem Bimetall-Salzkomplex, Triphenylbor enthält.

7. Ein Verfahren nach Anspruch 6, wobei das flüssige Sorptionsmittel 2 bis 5 Molprozent, bezogen auf das Kupfer in dem Bimetall-Salzkomplex, Triphenylbor enthält.

8. Ein Verfahren nach einem der Ansprüche 1 bis 7, wobei weniger als 50% des Zinkphenyl oder Triphenylbor am Beginn des Liganden-Abtrennungsverfahrens anwesend sind und der Rest absatzweise oder kontinuierlich während des Verfahrens der Ligandenabtrennung zugesetzt wird.